# EUROPEAN PATENT APPLICATION

(11) **EP 3 150 618 A1**
(43) Date of publication of application: **05.04.2017**
(21) Application number: 15306526.3
(22) Date of filing: 29.09.2015
(51) Int. Cl.: C07H 17/08, C07H 1/00

(54) **PROCESS FOR THE PREPARATION OF SELAMECTIN**

(71) Applicant: VIRBAC, 06516 Carros (FR)
(72) Inventor: MOUTOU, Jean-Luc, 06800 Cagnes-sur-mer (FR); GUIGO, Agnès, 06100 Nice (FR); SURENDRA MOHILE, Swapnil, 400601 Maharashtra (IN); MAHADEV KINAGE, Yogesh, 411026 Maharashtra (IN)
(74) Representative: Gevers & Orès

(57) **Abstract**

The invention relates to an improved process for the preparation of selamectin of formula 5, and intermediates following two routes:

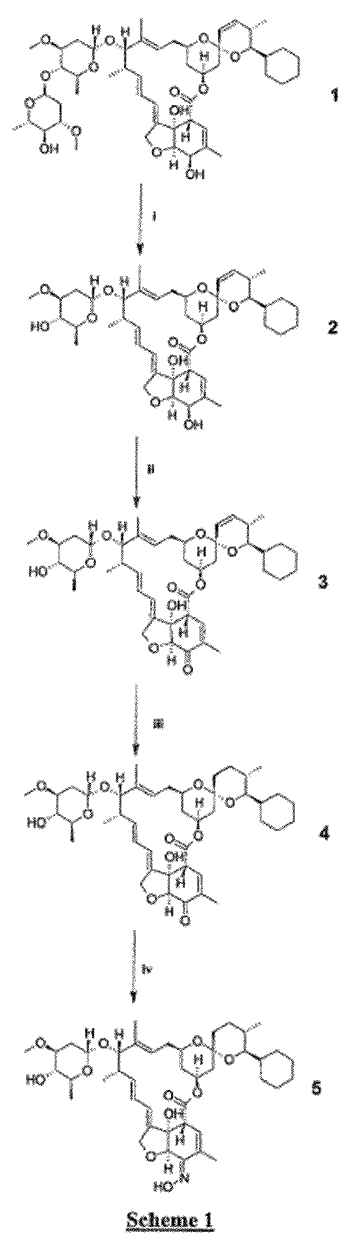

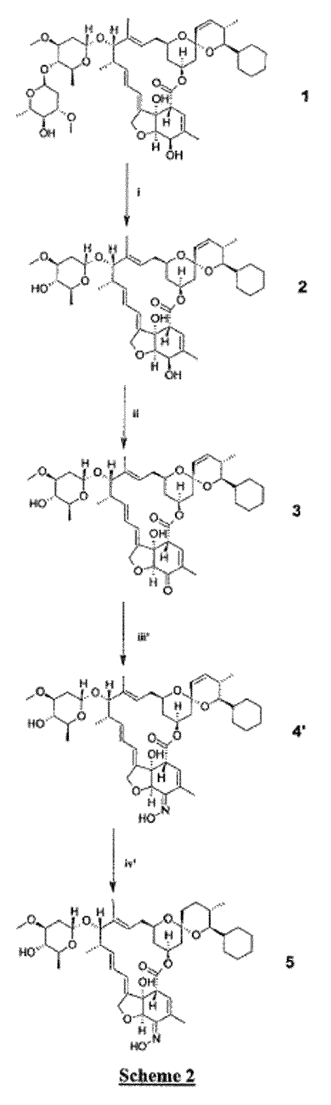

## Description

The invention relates to an improved process for the preparation of selamectin, and intermediates thereof.

(5Z,25S)-25-cyclohexyl-4'-O-de(2,6-dideoxy-3-O-methyl-α-L-arabino-hexopyranosyl)-5-demethoxy-25-de(1-methylpropyl)-22,23-dihydro-5-hydroxyiminoavermectin A₁ₐ, or (10E,14E,16E,21Z)-(1R,4S,5'S,6R,6'S,8R,12S,13S,20R,21R,24S)-6'-cyclohexyl-24-hydroxy-21-hydroxyimino-5',11,13,22-tetramethyl-2-oxo-(3,7,19-trioxatetracyclo[15.6.1.1^{4,8}.0^{20,24}]pentacosa-10,14,16,22-tetraene)-6-spiro-2'-(tetrahydropyran)-12-yl 2,6-dideoxy-3-O-methyl-α-L-arabino-hexopyranoside, also known as selamectin, is a topical anti-parasitic and anti-helminthic agent used on dogs and cats for preventing and treating infections of heartworms, fleas, ear mites, sarcoptic mange (scabies), and certain types of ticks in dogs, and prevents heartworms, fleas, ear mites, hookworms, and roundworms in cats. Selamectin is also effective against other nematodes which can infest companion animals including, for example, Dirofilaria in dogs and various parasites including gastro-intestinal parasites such as Ancylostoma, Necator, Ascaris, Strongyloides, Trichinella, Capillaria, Toxocara, Toxascaris, Trichuris, Enterobius, and the extraintestinal stages of Strongyloides, Towocara and Trichinella.

The basic process synthesis of selamectin is described in US 5,981,500, this process comprising successively the steps of hydrogenation of doramectin using Wilkinson's catalyst, desugaring using sulphuric acid, oxidation in the presence of manganese dioxide and oximation in anhydrous pyridine using hydroxylamine hydrochloride.

An improved process of the basic process is described in WO99/07721 and US 6,906,184, where the intermediate of the oxidation product is reacted with hydroxylamine hydrochloride to produce 5-oxime and the hydrolysis step for producing monosaccharide derivatives are conducted as a single simultaneous reaction.

These processes have the main disadvantage of using manganese dioxide generating effluent which may have difficulties in disposal on large scale, which renders the industrial production difficult. In addition, manganese dioxide has to be activated by removing water azeotropicaly in toluene (J. Org. Chem., 1969, 34 (6), 1979-1981), which leads to long reaction time and is unfavorable to increase the efficiency of the reaction.

WO2013/149577 provides an alternative synthesis process for obtaining selamectin via hydrogenation in the presence of Wilkinson's catalyst, oxidation with Dess-Martin periodinane, oximation and desugaring.

However, all these processes use a high quantity of Wilkinson's catalyst in their first stage, said catalyst being particularly expensive.

The inventors have now surprisingly discovered a new route of synthesis of selamectin by performing the reaction sequence differently, and provide a new process of selamectin in which the selectivity and purity of the intermediates and final products is improved, and the cost of the global process is significantly reduced. The inventors have observed that the implementation of the hydrogenation reaction in a subsequent step considerably reduced the quantity of Wilkinson's catalyst used compared to the quantities used when it is implemented in a first step.

According to a first embodiment, the overall process for preparing (5Z,25S)-25-cyclohexyl-4'-O-de(2,6-dideoxy-3-O-methyl-a-L-arabino-hexopyranosyl)-5-demethoxy-25-de(1-methylpropyl)-22,23-dihydro-5-hydroxyiminoavermectin A₁ₐ, also known as selamectin, comprises the steps of:
(i) deglycosidation of 25-cyclohexyl-5-O-demethyl-25-de(1-methylpropyl)avermectin A₁ₐ, also known as doramectin (Compound **1**), in an organic solvent, with a strong acid, and preferably p-tolenesulfonic acid (p-TSA) or sulfuric acid (H₂SO₄), to obtain Compound **2** (IUPAC name: Avermectin A1a, 25-cyclohexyl-4'-O-de(2,6-dideoxy-3-O-methyl-α-L-arabino-hexopyranosyl)-5-o-demethyl-25-de(1-methylpropyl)-(9Cl)),
(ii) oxidation of Compound **2** with Dess-Martin periodinane (DMP), to obtain Compound **3** (IUPAC name: Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl)),
(iii) catalytic hydrogenation of Compound **3**, to obtain Compound **4** (IUPAC name: Avermectin A1a, 25-cyclohexyl-4'-O-de(2,6-dideoxy-3-O-methyl-α-L-arabino-hexopyranasyl)-5-demethoxy-25-de(1-methylpropyl)-22,23-dihydro-5-oxo-(9Cl)), preferably using Wilkinson's catalyst (tris(triphenylphosphine)rhodium (I) chloride),
(iv) oximation of Compound **4** with hydroxylamine hydrochloride in an organic solvent, preferably using water and an organic solvent, or in a mixture of organic solvents miscible in water, to obtain selamectin (Compound **5**),
(v) optionally, crystallizing selamectin (Compound **5**).

The reaction scheme for preparing selamectin according to this first embodiment is shown in the **Scheme 1** below:

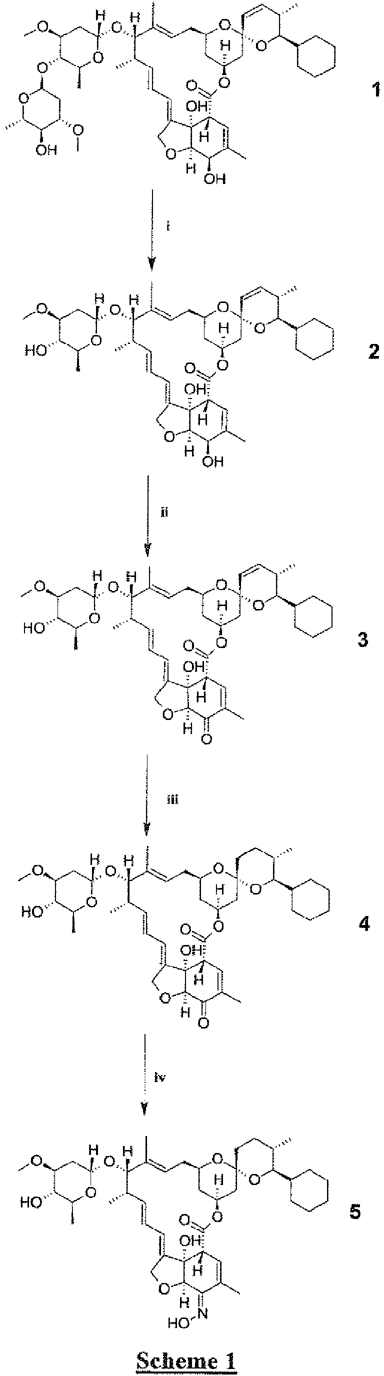

The deglycosidation of doramectin in a first step has the unexpected advantage of reducing the molecular weight, and therefore the contribution of expensive reagents in the rest of the reaction.

Alternatively, the steps (iii) and (iv) described above can be switched into steps (iii') and (iv'). According to this second embodiment, the overall process for preparing (5Z,25S)-25-cyclohexyl-4*-O-de(2,6-dideoxy-3-O-methyl-α-L-arabino-hexopyranosyl)-5-demethoxy-25-de(1-methylpropyl)-22,23-dihydro-5-hydroxyiminoavermectin A₁ₐ, also known as selamectin, comprises the steps of:
(i) deglycosidation of 25-cyclohexyl-5-O-demethyl-25-de(1-methylpropyl)avermectin A1a, also known as doramectin (Compound **1**), in an organic solvent, with a strong acid, and preferably p-tolenesulfonic acid (p-TSA) or sulfuric acid (H₂SO₄), to obtain Compound **2** (IUPAC name: Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl)),
(ii) oxidation of Compound **2** with Dess-Martin periodinane (DMP), to obtain Compound **3** (IUPAC name: Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl)),
(iii') oximation of Compound **3** with hydroxylamine hydrochloride in an organic solvent, to obtain Compound **4'** (IUPAC name: Avermectin A1a, 25-cyclohexyl-4'-O-de(2,6-dideoxy-3-O-methyl-α-L-arabino-hexopyranosyl)-5-demethoxy-25-de(1-methylpropyl)-5-(hydroxyimino)-(9Cl)), preferably using water and an organic solvent, or a mixture of organic solvents miscible in water,
(iv') catalytic hydrogenation of Compound **4'**, to obtain selamectin (Compound **5**), preferably using Wilkinson's catalyst (tris(triphenylphosphine)rhodium (I) chloride),
(v) optionally, crystallizing selamectin (Compound **5**).

The reaction scheme for preparing selamectin according to this second embodiment is shown in the **Scheme 2** below:

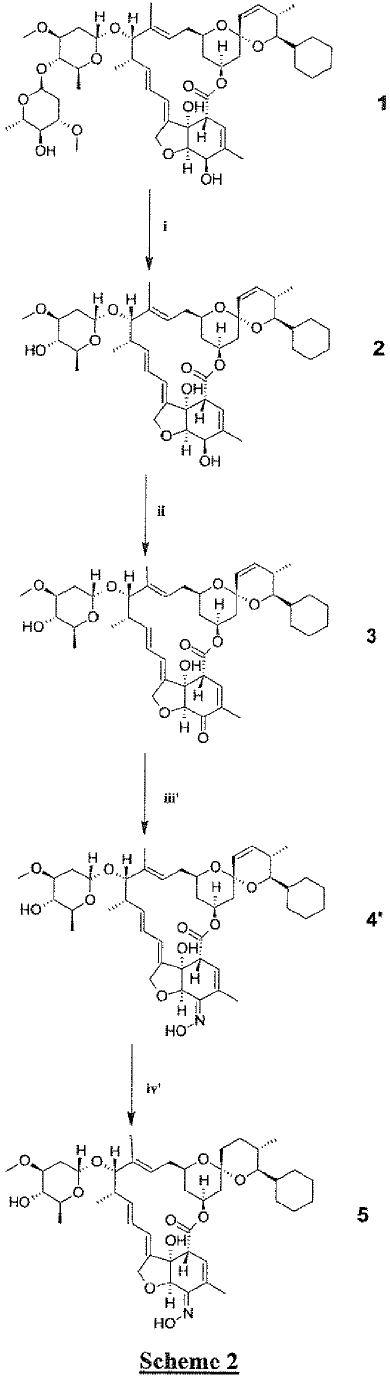

An objective of the present invention is also to provide novel methods for preparing selamectin intermediates.

According to an aspect, the invention aims to a process for the preparation of selamectin, which comprises reacting Compound **4** with hydroxylamine hydrochloride in water and in an organic solvent, or in a mixture of organic solvents miscible in water. Advantageously, this process is performed in a mixture of methanol, 1,4-dioxane and water. Said process has the advantages of being very clean and complete with very few impurities.

Said reaction of Compound **4** with hydroxylamine hydrochloride corresponds to step (iv) of the first overall process described in **Scheme 1**.

The reaction of Compound **4** with hydroxylamine hydrochloride may be performed at a temperature of 20-50°C, preferably of 20-30°C, and more preferably at around 25°C. Said reaction is generally complete after a period of 6-12 hours.

In the sense of the invention, the term "around" means 20%, preferably 10%, and more preferably 5% of the concerned value.

The reaction of Compound **4** with hydroxylamine hydrochloride is preferably carried out without any base.

According to another aspect, the invention aims to a process for the preparation of selamectin, which comprises hydrogenating Compound **4'**.

Said hydrogenation of Compound **4'** corresponds to step (iv') of the second overall process described in **Scheme 2**.

Advantageously, the hydrogenation of Compound **4'** is a homogeneous catalytic hydrogenation, preferably using Wilkinson's catalyst (tris(triphenylphosphine)rhodium (I) chloride) in a solvent. Ideally, said solvent is chosen among aromatic solvents or ketones, and more advantageously the aromatic solvent is toluene and the ketone is acetone. Advantageously, the molar ratio catalyst/Compound **4'** is of 0.01-0.5, preferably of 0.1-0.2, and more preferably is of 0.10, 0.11, 0.12, 0.13, 0.14, 0.15, 0.16, 0.17, 0.18, 0.19 or 0.20.

The hydrogenation of Compound **4'** is preferably performed at a pressure of 2-10 bar, preferably 3-5 bar. Advantageously, said hydrogenation step is carried out at a temperature of 40-60°C, and preferably at around 50°C. Said hydrogenation step is generally complete after a period of 1-6 hours, preferably of 1-3 hours, and more preferably of around 2 hours.

Preferably, the selamectin obtained according to step (iv) or (iv') described above is further purified by crystallization with a solvent. The obtained crystallized selamectin may be further crystallized one, two, or more times, with suitable solvents such as alcohols in C₁-C₆, esters in C₂-C₈, crown ethers in C₃-C₈, aromatic solvents in C₅-C₁₆, or aliphatic solvents in C₁-C₆, optionally substituted with halogen, hydroxyl, cyano, nitro, or amino groups, the preferred solvents being selected from methanol, ethanol, propanol, isopropanol, butanol, ethyl acetate, acetonitrile, dioxane, toluene, and mixture thereof, and optionally in mixture with water.

According to another aspect, the invention aims to a process for the preparation of Compound **4**, which comprises hydrogenating Compound **3**.

Said hydrogenation of Compound **3** corresponds to step (iii) of the first overall process described in **Scheme 1**.

Advantageously, the hydrogenation of Compound **3** is a homogeneous catalytic hydrogenation, preferably using Wilkinson's catalyst (tris(triphenylphosphine)rhodium (I) chloride) in a solvent. Ideally, said solvent is chosen among aromatic solvents or ketones, and more advantageously the aromatic solvent is toluene and the ketone is acetone. Advantageously, the molar ratio catalyst/Compound 3 is of 0.01-0.5, preferably of 0.1-0.2, and more preferably is of 0.10, 0.11, 0.12, 0.13, 0.14, 0.15, 0.16, 0.17, 0.18, 0.19 or 0.20.

The hydrogenation of Compound **3** is preferably performed at a pressure of 2-10 bar, preferably 3-5 bar. Advantageously, said hydrogenation step is carried out at a temperature of 40-60°C, preferably at around 50°C. Said hydrogenation step is generally complete after a period of 1-3 hours, preferably of around 2 hours.

According to another aspect, the invention aims to a process for the preparation of Compound **4'**, which comprises reacting Compound 3 with hydroxylamine hydrochloride in water and in an organic solvent, or a mixture of organic solvents miscible in water. Advantageously, this process is performed in a mixture of methanol, 1,4-dioxane and water. Said process has the advantages of being very clean and complete with very few impurities.

Said reaction of Compound **3** with hydroxylamine hydrochloride corresponds to step (iii') of the second overall process described in **Scheme 2**.

The reaction of Compound **3** with hydroxylamine hydrochloride may be performed at a temperature of 20-50°C, preferably of 20-30°C, and more preferably at around 25°C. Said reaction is generally complete after a period of 6-12 hours.

In the sense of the invention, the term "around" means 20%, preferably 10%, and more preferably 5% of the concerned value.

The reaction of Compound **3** with hydroxylamine hydrochloride is preferably carried out without any base.

According to another aspect, the invention aims to a process for the preparation of Compound 3, which comprises oxidizing Compound **2** with Dess-Martin periodinane (DMP), preferably in a range from 1 to 2.5 equivalents, and more preferably at 1.2 equivalent.

Said oxidation of Compound **2** corresponds to step (ii) of the first and second overall processes described in **Schemes 1** and **2**.

Advantageously, the oxidation is performed in an aprotic organic solvent, preferably chosen among dichloromethane, chloroform, methyl tert-butylether (MTBE), acetonitrile, or a mixture thereof.

Preferably, the oxidation is performed at a temperature of 0-40°C. After completion of reaction, reagent is quenched in 10% sodium thiosulphate solution. The reaction time is adjusted to obtain a mono-oxidized Compound **3**. Said oxidation step is generally complete after a period of 2-4 hours, more preferably of around 3 hours.

According to another aspect, the invention aims to a process for the preparation of Compound **2**, which comprises the reaction of deglycosidation of doramectin (Compound **1**) with a strong acid.

Said deglycosidation step of doramectin corresponds to step (i) of the first and second overall processes described in **Schemes 1** and **2**.

Advantageously, the strong acid used during the deglycosidation step of doramectin is an inorganic acid such as sulfuric acid (H₂SO₄), hydrochloric acid (HCl), phosphoric acid (H₃PO₄), or an organic acid, such as trifluoroacetic acid (TFA), p-tolenesulfonic acid (p-TSA), methanesulfonic acid (MSA), or acidic cation resins such as Dowex^{®} 50WX4, Diaion^{®} RCP-160M, Diaion^{®} PK 208LM. More preferably, the strong acid is sulfuric acid (H₂SO₄) or p-tolenesulfonic acid (p-TSA).

The deglycosidation of doramectin is performed in an organic solvent, and advantageously in an organic polar solvent, and more advantageously in a mixture of an organic polar solvent and water. Said organic polar solvent may be acetonitrile or an alcohol in C₁-C₆, such as ethanol. Particularly preferred acid/solvents couple used during the deglycosidation step of doramectin are p-tolenesulfonic acid (p- TSA)/ethanol, and sulfuric acid (H₂SO₄) in acetonitrile and water.

The temperature for the deglycosidation of doramectin is adjusted by the man skilled in the art depending on the acid selected. However, this step may preferably be performed at a temperature of 10-90°C, and more preferably when the strong acid is sulfuric acid (H₂SO₄) the temperature is of around 25°C, and when the acid is p-tolenesulfonic acid (p-TSA) the temperature is of around 80°C. The time of reaction of the deglycosidation step is adjusted by the man skilled in the art depending on the reagents used. However, this step may preferably last 1-8 hours, and more preferably it lasts 4 hours.

The obtained Compound **2** may be further purified by standard means, including column chromatography or crystallization.

In addition to the above provisions, the invention also comprises other provisions which will emerge from the examples which follow.

### EXAMPLES:

### Process description:

### 1) Preparation of Avermectin A1a, 25-cyclohexyl-4'-O-de(2,6-dideoxy-3-O-methyl-α-L-arabino-hexopyranosyl)-5-0-demethyl-25-de(1-methylpropyl)-(9Cl)

2.5 L of a 9/1 acetonitrile/water solution containing 2% (50 g) of sulfuric acid (H₂SO₄) are introduced in a 3 L reactor. 100 g of 25-cyclohexyl-5-O-deinethyl-25-de(1-methylpropyl)avermectin A1a are added and the mixture is stirred at room temperature for 4-5 hours. A saturated NaHCO₃ solution is added and the reaction mixture is stirred for 15 minutes. The reaction mixture is filtered and the filtrate is concentrate under vacuum to remove acetonitrile. Ethyl acetate is added and the mixture is stirred for 10-15 minutes. The organic layer is separated, washed with brine, and evaporated under vacuum to afford crude Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl) (88 g). Crude Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl) is purified by column chromatography to obtained 66.7 g (yield: 79%, purity 92.3%) of pure Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl).

C₄₃H₆₂O₁₁ (754.97 g/mol); m/z (-vemode): 753; mp: 147-149°C.

¹H NMR (MHz; CDCl₃): δ = 0.80-0.90 (m, 1H), 0.92 (d, 3H), 1.16 (d, 3H), 1.20-1.40 (m, 4H), 1.27 (d, 3H), 1.40-1.52 (m, 2H), 1.50 (s, 3H), 1.52-1.72 (m, 9H), 1.72-1.85 (m, 1H), 2.01 (dd, 1H), 2.04 (s, 3H), 2.20-2.40 (m, 2H), 2.42-2.58 (m, 1H), 3.17 (t, 1H), 3.32 (d, 1H), 3.39 (m, 1H), 3.48 (s, 3H), 3.50-3.60 (m, 1H), 3.80-3.90 (m, 2H), 3.92-4.05 (m, 2H), 4.25-4.35 (m, 1H), 4.70 (s, 2H), 4.83 (m, 1H), 5.00 (dd, 1H), 5.35-5.50 (m, 1H), 5.43 (s, 1H), 5.55 (d, 1H), 5.68-5.72 (m, 3H), 5.88 (d, 1H).

¹³C NMR (MHz, CDCl₃): 5 = 14.9, 16.3, 17.5, 19.7, 20.0, 25.3, 26.2, 26.3, 26.7, 29.7, 30.7, 31.2, 33.7, 34.1, 36.4, 38.4, 39.5, 40.1, 45.4, 56.3, 67.4, 67.8, 67.9, 68.2, 75.8, 77.2, 78.1, 78.9, 80.1, 81.3, 94.6, 95,4, 117.8, 117.9, 120.3, 124.4, 127.5, 134.7, 135.9, 137.7, 137.9,139.2,173.6.

### 2) Preparation of Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl)

1.34 L of dichloromethane, and 66.7 g of Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl) are charged in a 3 L reactor. After dissolution of Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl), the reaction mass is cooled to 0-5°C. 56.2 g of Dess-Martin periodinane (DMP) is added and the reaction mass is stirred at 0-25°C. The reaction is monitored after every 1 hour by thin-layered chromatography (TLC) until complete consumption of Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl).

Then, the reaction mixture is filtered through a celite bed and washed with dichloromethane. 335 mL of 10% aqueous sodium thiosulphate (Na₂S₂O₃) solution are added to the filtrate and the mixture is stirred for 30 minutes. The organic layer is collected and the aqueous layer is extracted a second time with dichloromethane. The organic layers are combined and successively washed with 1 N aqueous HCl solution, 10% aqueous of sodium bicarbonate (NaHCO₃) solution and brine. The organic layer is distilled off under reduced pressure to afford 65.8 g (yield: 100%; HPLC purity: 85.0%) of crude Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl) as a yellow solid.

C₄₃H₆₀O₁₁ (752.95 g/mol); m/z (-ve mode): 751; mp: 129-131°C.

¹H NMR (MHz; CDCl₃): δ = 0.80-0.89 (m, 1H), 0.94 (d, 3H), 1.3-1.45 (m, 1H), 1.16 (d, 3H), 1.28 (d, 3H), 1.51 (s, 3H), 1.52-1.60 (m, 1H), 1.70-1.80 (m, 1H), 1.90 (s, 3H), 2.00-2.1 (m, 1H), 2.20-2.40 (m, 3H), 2.5-2.6 (m, 1H), 3.10-3.20 (m, 1H), 3.5 (s, 3H), 3.55-3.60 (m, 1H), 3.80-3.90 (m, 3H), 3.97 (s, 1H), 4.65 (s, 2H), 5.00 (dd, 1H), 4.8-4.9 (m, 1H), 5.40-5.50 (m, 1H), 5.54 (d, 1H), 5.65-5.90 (m, 3H), 5.98 (d, 1H), 6.60 (d, 1H).

¹³C NMR (MHz, CDCl₃): δ = 15.2, 15.5, 16.6, 17.7, 20.1, 25.6, 26.5, 26.6, 27.0, 30.0, 31.5, 34.0, 34.3, 36.6, 38.6, 39.8, 40.4, 46.6, 56.6, 68.1, 68.2, 69.0, 69.8, 76.0, 77.4, 78.4, 80.9, 81.5, 82.1, 95.0, 95.8, 118.1, 122.0, 124.6, 127.6, 135.3, 136.3, 137.8, 138.0, 138.3, 139.2, 172.2,192.3.

The crude Avermectin A1a, 25-cyclohexyl-4'-O-de(2,6-dideoxy-3-O-Methyl-α-L-arabino-hexopyranosyl)-5-demethoxy-25-de(1-methylpropyl)-5-oxo-(9Cl) is taken as such for next stage.

### 3) Preparation of Avermectin A1a, 25-cyclohexyl-4'-O-de(2,6-dideoxy-3-O-methyl-α-L-arabino-hexopyranosyl)-5-demethoxy-25-de(1-methylpropyl)-22,23-dihydro-5-oxo-(9Cl)

65.8 g of Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl), 1.316 L of toluene, and 12.9 g of Wilkinson's catalyst are charged in a dry and clean 2 L autoclave. The autoclave is closed, flushed with N₂ and charged with hydrogen to 3.5 bar. The reaction mixture is stirred at 50°C for 2 hours maintaining the hydrogen pressure at 3.5 bar.

Then, the reaction mixture is cooled to 25-30°C and the reactor is flushed with N₂ to remove hydrogen. The reaction mass is filtered and the filtrate is concentrated by half under reduce pressure. Then, the reaction mixture is filtered on filter aid to remove the catalyst, and the filter aid is washed with toluene. The filtrates are combined and distilled off under reduced pressure to give 68.0 g (yield: 100%; HPLC purity: 83.3%) of crude Avermectin A1a, 25-cyclohexyl-4'-O-de(2,6-dideoxy-3-O-methyl-α-L-arabino-hexopyranosyl)-5-demethoxy-25-de(1-methylpropyl)-22,23-dihydro-5-oxo-(9Cl) as a brown solid.

C₄₃H₆₂O₁₁ (754.97 g/mol); m/z (-ve mode): 753; mp: 108-110°C.

¹H NMR (MHz; CDCl₃): δ = 0.80 (d, 3H), 0.82-0.92 (m, 1H), 1.16 (d, 3H), 1.20-1.72 (m, 18 H), 1.28 (d, 3H), 1.51 (s, 3H), 1.72-1.86 (m, 1H), 1.90 (s, 3H), 2.00 (dd, 1H), 2.18-2.40 (m, 3H), 2.48-2.60 (m, 1H), 3.18 (dd, 1H), 3.47 (s, 2H), 3.52-3.62 (m, 2H), 3.62-3.70 (m, 1H),3.80-3.90 (m, 1H), 3.97(s, 1H), 4.03 (s, 1H), 4.75 (s, 2H), 4.83 (dd, 1H), 4.98 (dd, 1H), 5.40-5.50 (m, 1H), 5.65-5.80 (m, 2H), 5.91-6.00 (m, 1H), 6.59 (s, 1H).

¹³C NMR (MHz, CDCl₃): δ = 15.2, 15.5, 17.5, 17.8, 20.1, 24.7, 26.6, 27.0, 28.1, 29.7, 30.7, 34.0, 34.1, 35.7, 37.0, 38.6, 39.9, 41.1, 46.6, 56.6, 67.1, 68.1, 69.3, 69.9, 76.1, 78.3, 78.6, 80.8, 81.6, 81.9, 95.0, 118.3, 122.0, 124.6, 128.5, 132.1, 135.0, 136.8, 137.8, 138.0, 139.2, 172.03, 192.2.

The crude Avermectin A1a, 25-cyclohexyl-4'-O-de(2,6-dideoxy-3-O-methyl-α-L-arabino-hexopyranosyl)-5-demethoxy-25-de(1-methylpropyl)-22,23-dihydro-5-oxo-(9Cl) is taken as such for next stage.

### 4) Preparation of Avermectin A1a, 25-cyclohexyl-4'-O-de(2,6-dideoxy-3-O-methyl-α-L-arabino-hexopyranosyl)-5-demethoxy-25-de(1-methylpropyl)-5-hydroxyimino-(9Cl)

120 mL of a 1/1 mixture of methanol and 1,4-dioxane, and 5 g of Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl), are charged in a 250 mL reactor. After dissolution of Avermectin A1a, 25-cyclohexyl-4'-O-de(2,6-dideoxy-3-4-methyl-α-L-arabino-hexopyranosyl)-5-demethoxy-25-de(1-methylpropyl)-5-oxo-(9Cl), the reaction mass is cooled to 0-5°C. A solution of hydroxylamine hydrochloride (4.15 g) in water is slowly added to the reaction mass and stirred at 25-30°C for 12 hours.

The reaction mixture is evaporated to remove methanol and dioxane. 15 mL of water and 15 mL of methyl tert-butyl ether (MTBE) are added to the concentrate and stirred for 30 min. The organic layer is collected and the aqueous layer is extracted with methyl tert-butyl ether (MTBE). The organic layers are combined and distilled off under reduced pressure to afford 5.7 g (HPLC purity: 86.9%) of crude product. This crude product is crystallized in acetonitrile to afford 3.1 g (yield: 61 %, HPLC purity: 95.5%) of pure Avermectin A1a, 25-cyclohexyl-4'-O-de(2,6-dideoxy-3-O-methyl-α-L-arabino-hexopyranosyl)-5-demethoxy-25-de(1-methylpropyl)-5-hydroxyimino-(9Cl).

C₄₃H₆₃NO₁₁ (767.95 g/mol); m/z (-ve mode) 767; mp: 206-208°C.

¹H NMR (MHz; CDCl₃): δ = 0.93 (d, 3H), 0.80-0.90 (m, 1H), 1.16 (d, 3H) 1.20-1.40 (m, 4H), 1.27 (d, 3H), 1.50 (s, 3H), 1.40-1.65 (m, 6H), 1.65-1.90 (m, 5H), 1.94 (s, 3H), 2.20-2.40 (m, 4H), 2.50-2.60 (m, 1H), 3.07 (b, 1H), 3.18 (t, 1H), 3.32 (d, 1H), 3.42 (s, 1H), 3.50 (s, 3H), 3.55-3.65 (m, 1H), 3.80-4.05 (m, 4H), 4.68 (s, 1H), 4.60-4.80 (m, 2H), 4.84 (s, 1H), 4.90-5.10 (m, 1H), 5.35-5.50 (m, 1H), 5.50-5.60 (m, 1H), 5.70-5.80 (m, 3H), 5.83 (s, 1H), 5.90-6.00 (m, 1H), 9.39 (s, 1H).

¹³C NMR (MHz, CDCl₃): δ =15.2, 16.7, 17.6, 17.8, 20.2, 25.6, 26.5, 26.6, 27.0, 30.0, 31.4, 34.0, 34.3, 36.7, 38.7, 39.9, 40.4, 46.5, 56.7, 68.1, 68.3, 68.5, 68.8, 73.0, 75.9, 77.4, 78.6, 78.7, 81.7, 94.9, 95.8, 118.1, 121.6, 124.8, 125.4, 127.7, 132.0, 135.1, 136.3, 138.0, 138.4, 151.4, 173.1.

### 5) Preparation of selamectin.

### Process 1 (1^{st} embodiment):

1.6 L of a 1/1 mixture of methanol and 1,4-dioxane, and 68 g of Avermectin A1a, 25-cyclohexyl-4'-O-de(2,6-dideoxy-3-O-methyl-α-L-arabino-hexopyranosyl)-5-demethoxy-25-de(1-methylpropyl)-22,23-dihydro-5-oxo-(9Cl), are charged in a 3 L reactor. After dissolution of Avermectin A1a, 25-cyclohexyl-4'-O-de(2,6-dideoxy-3-O-methyl-α-L-arabino-hexopyranosyl)-5-demethoxy-25-de(1-methylpropyl)-22,23-dihydro-5-oxo-(9Cl), the reaction mass is cooled to 0-5°C. A solution of hydroxylamine hydrochloride (56.3 g) in water is slowly added to the reaction mass and stirred at 25-30°C for 12 hours.

Then, 3.4 g of charcoal is added to the reaction mass and stirred for 1 hour at room temperature. The suspension is filtered over filter aid and the filter aid is washed with methanol. The filtrate is evaporated to remove methanol and dioxane. 2 L of water and 2 L of methyl tert-butyl ether (MTBE) are added to the concentrate and stirred for 30 min. The organic layer is collected and the aqueous layer is extracted with MTBE. The organic layers are combined and distilled off under reduced pressure to afford 77 g (yield: 100%, HPLC purity 86.9%) of crude selamectin.

### Process 2 (2^{nd} embodiment):

1.6 g of Avermectin A1a, 25-cyclohexyl-4'-O-de(2,6-dideoxy-3-O-methyl-α-L-arabino-hexopyranosyl)-5-demethoxy-25-de(1-methylpropyl)-5-hydroxyimino-(9Cl), 80 mL of acetone, and 0.192 g of Wilkinson's catalyst are charged in a dry 400 mL autoclave. The autoclave is closed, flushed with N₂ and charged with hydrogen to 3.5 bar. The reaction mixture is stirred at 50°C for 6 hours maintaining the hydrogen pressure at 3.5 bar.

Then, the reaction mixture is cooled to 25-30 °C and the reactor is flushed with N₂ to remove hydrogen. Charcoal (80 mg, 5% w/w) is added and the mixture is stirred at 25-30°C for one hour. Then, the reaction mixture is filtered on filter aid, and the filter aid is washed with acetone. The filtrates are combined and distilled off under reduced pressure to give 1.8 g (yield: 100%; HPLC purity: 91.9%) of crude selamectin as a brown oil.

### 6) Crystallization step

### Process 1 (1^{st} embodiment):

4 mL of toluene and 1 g of crude selamectin are heated to 75-80°C for 1 hour. The heating is stopped and the reaction mass cooled to 25-30°C. The reaction mass is then maintained at 10°C for 3 hours, and filtered. The wet cake is washed with toluene and dried to give 0.52 g (yield: 52%, HPLC purity: 96.7%) of pure selamectin.

### Process 2 (2^{nd} embodiment):

4.5 mL of acetonitrile and 0.9 g of crude selamectin are stirred at room temperature for 1 hour. The reaction mass is then cooled to 0°C for 1 hour to allow the product to crystallize. The reaction media is filtered, the wet cake is washed with cold acetonitrile and dried to give 0.48 g (yield: 53%, HPLC purity: 96.2%) of pure selamectin.

C₄₃H₆₃NO₁₁ (769.95 g/mol); m/z (-ve mode): 769; mp: 208-210°C.

¹H NMR (MHz; CDCl₃): δ = 0.78 (d, 3H), 0.82-0.90 (m, 1H), 1.16 (s, 3H), 1.20-1.80 (m, 14H), 1.27 (d, 3H), 1.32-1.70 (m, 3H), 1.33-1.45 (m, 1H), 1.51 (s, 3H), 1.70-1.85 (m, 1H), 1.90-2.05 (m, 1H), 1.95 (s, 3H), 2.16-2.40 (m, 3H), 2.45-2.58 (m, 1H), 3.08 (d, 1H), 3.18 (t, 1H), 3.37-3.45 (m, 1H), 3.48 (s, 3H), 3.52-3.60 (m, 1H), 3.60-3.70 (m, 1H), 3.82-3.90 (m, 1H), 3.93-4.02 (m, 1H), 4.68 (s, 1H), 4.70-4.78 (s, 2H), 4.83 (d, 1H), 4.97 (dd, 1H), 5.48-5.50 (m, 1H), 5.68-5.90 (m, 2H), 5.84 (s, 1H), 5.96 (d, 1H).

¹³C NMR (MHz, CDCl₃): δ = 15.4, 17.6, 17.6, 17.9, 20.16, 24.7, 26.5, 26.9, 28.0, 29.6, 30.6, 31.2, 33.9, 34.1, 35.6, 36.9, 38.5, 39.8, 41.1, 46.4, 56.6, 67.1, 68.1, 68.7, 68.8, 72.9, 75.9, 78.4, 78.5, 78.8, 81.7, 94.9, 97.7, 118.2, 121.5, 124.7, 125.4, 132.2, 135.1, 138.2, 138.4, 151.7, 173.4.

## Claims

1. A process for the preparation of (5Z,25S)-25-cyclobexyl-4'-O-de(2,6-dideoxy-3-O-methyl-α-L-arabino-hexopyranosyl)-5-demethoxy-25-de(1-methylpropyl)-22,23-dihydro -5-hydroxyiminoavermectin A₁ₐ, which comprises the steps of:
(i) deglycosidation of 25-cyclohexyl-5-O-demethyl-25-de(1-methylpropyl) avermectin A1a, in an organic solvent, with a strong acid, to obtain Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl),
(ii) oxidation of Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl) with Dess-Martin periodinane (DMP), to obtain Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl),
(iii') oximation of Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl), to obtain Avermectin A1a, 25-cyclohexyl-4'-O-de(2,6-dideoxy-3-4-methyl-α-L-arabino-hexopyranosyl)-5-demethoxy-25-de(1-methylpropyl)-5-(hydroxyimino)-(9Cl)), preferably using water and an organic solvent, or a mixture of organic solvents miscible in water,
(iv') catalytic hydrogenation of Avermectin A1a, 25-cyclohexyl-4'-O-de(2,6-dideoxy-3-O-methyl-α-L-arabino-hexopyranosyl)-5-demethoxy-25-de(1-methylpropyl)-5-(hydroxyimino)-(9Cl)), to obtain (5Z,25S)-25-cyclohexyl-4'-O-de(2,6-dideoxy-3-O-methyl-α-L-arabino-hexopyranosyl)-5-demethoxy-25-de(1-methylpropyl)-22,23-dihydro-5-hydroxyiminoavermectin A1a,
(v) optionally, crystallizing (5Z,25S)-25-cyclohexyl-4'-O-de(2,6-dideoxy-3-O-methyl-α-L-arabino-hexopyranosyl)-5-demethoxy-25-de(1-methylpropyl)-22,23-dihydro-5-hydroxyiminoavermectin A1a.

2. A process for the preparation of (5Z,25S)-25-cyclohexyl-4'-O-de(2,6-dideoxy-3-O-methyl-α-L-arabino-hexopyranosyl)-5-demethoxy-25-de(1-methylpropyl)-22,23-dihydro-5-hydroxyiminoavermectin A1a, which comprises hydrogenating Avermectin A1a, 25-cyclohexyl-4'-O-de(2,6-dideoxy-3-O-methyl-α-L-arabino-hexopyranosyl)-5-demethoxy-25-de(1-methylpropyl)-5-(hydroxyimino)-(9Cl)).

3. A process as claimed in Claim 2, wherein the hydrogenation is a homogeneous catalytic hydrogenation using Wilkinson's catalyst in a solvent, said solvent being preferably chosen among toluene or acetone.

4. A process as claimed in Claim 2 or Claim 3, wherein the hydrogenation is performed at a pressure of 2-10 bar, preferably at a temperature of 40-60°C.

5. A process as claimed in Claims 2 to 4, wherein the obtained (5Z,25S)-25-cyclohexyl-4'-O-de(2,6-dideoxy-3-O-methyl-α-L-arabino-hexopyranosyl)-5-demethoxy-25-de(1-methylpropyl)-22,23-dihydro-5-hydroxyiminoavermectin A₁ₐ is further purified by crystallization with a solvent.

6. A process for the preparation of Avermectin A1a, 25-cyclohexyl-4'-O-de(2,6-dideoxy-3-Omethyl-α-L-arabino-hexopyranosyl)-5-demethoxy-25-de(1-methylpropyl)-5-(hydroxyimino)-(9Cl)), which comprises reacting Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl) with hydroxylamine hydrochloride in water and in an organic solvent, or in a mixture of organic solvents miscible in water.

7. A process as claimed in Claim 6, wherein the solvent is a mixture of methanol, 1,4-dioxane and water.

8. A process as claimed in Claim 6 or Claim 7, wherein the process is performed at a temperature of 20-50°C, and preferably of 20-30°C.

9. A process for the preparation of: (5Z,25S)-25-cyclohexyl-4'-O-de(2,6-dideoxy-3-O-methyl-α-L-arabino-hexopyranosyl)-5-demethoxy-25-de(1-methylpropyl)-22,23-dihydro-5-hydroxyiminoavermectin A₁ₐ, which comprises the steps of:
(i) deglycosidation of 25-cyclohexyl-5-O-demethyl-25-de(1-methylpropyl)avermectin A₁ₐ, in an organic solvent, with a strong acid, to obtain Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl),
(ii) oxidation of Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl) with Dess-Martin periodinane (DMP), to obtain Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl),
(iii) catalytic hydrogenation of Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl), to obtain Avermectin A1a, 25-cyclohexyl-4'-O-de(2,6-dideoxy-3-O-methyl-α-L-arabino-hexopyranosyl)-5-demethoxy-2S-de(1-methylpropyl)-22,23-dihydro-5-oxo-(9Cl),
(iv) oximation of Avermectin A1a, 25-cyclohexyl-4'-O-de(2,6-dideoxy-3-O-methyl-α-L-arabino-hexopyranosyl)-5-demethoxy-25-de(1-methylpropyl)-22,23-dihydro-5-oxo-(9Cl) with hydroxylamine hydrochloride in an organic solvent, or in a mixture of organic solvents miscible in water, to obtain (5Z,25S)-25-cyclohexyl-4'-O-de(2,6-dideoxy-3-O-methyl-α-L-arabino-hexopyranosyl)-5-demethoxy-25-de(1-methylpropyl)-22,23-dihydro-5-hydroxyiminoavermectin A₁ₐ,
(v) optionally, crystallizing (5Z,25S)-25-cyclohexyl-4'-O-de(2,6-dideoxy-3-O-methyl-α-L-arabino-hexopyranosyl)-5-demethoxy-25-de(1-methylpropyl)-22,23-dihydro-5-hydroxyiminoavermectin A₁ₐ.

10. A process for the preparation of (5Z,25S)-25-cyclohexyl-4'-4-de(2,6-dideoxy-3-O-methyl-α-L-arabino-hexopyranosyl)-5-demethoxy-25-de(1-methylpropyl)-22,23-dihydro-5-hydroxyiminoavermectin A₁ₐ, which comprises reacting Avermectin A1a, 25-cyclohexyl-4'-O-de(2,6-dideoxy-3-O-methyl-α-L-arabino-hexopyranosyl)-5-demethoxy-25-de(1-methylpropyl)-22,23-dihydro-5-oxo-(9Cl) with hydroxylamine hydrochloride in water and in an organic solvent, or in a mixture of organic solvents miscible in water.

11. A process for the preparation of Avermectin A1a, 25-cyclohexyl-4'-O-de(2,6-dideoxy-3-D-methyl-α-L-arabino-hexopyranosyl)-5-demethoxy-25-de(1-methylpropyl)-22,23-dihydro-5-oxo-(9CI), which comprises hydrogenating Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl).

12. A process for the preparation of Avermectin A1a, 25-cyclohexyl-4'-O-de(2,6-dideoxy-3-O-methyl-α-L-arabino-hexopyranosyl)-5-demethoxy-25-de(1-methylpropyl)-5-oxo-(9Cl), which comprises oxidizing Avermectin A1a, 25-cyclohexyl-4'-O-de(2,6-dideoxy-3-O-methyl-α-L-arabino-hexopyra-nosyl)-5-O-demethyl-25-de(1-methylpropyl)-(9Cl) with Dess-Martin periodinane (DMP).

13. A process as claimed in Claim 12, wherein the oxidation is performed in an aprotic organic solvent, preferably chosen among dichloromethane, chloroform, methyl tert-butyl ether (MTBE), acetonitrile, or a mixture thereof.

14. A process as claimed in Claim 12 or Claim 13, wherein the oxidation is performed at a temperature of 0-40°C, preferably during a period of 2-4 hours.

15. A process for the preparation of Avermectin A1a, 25-cyclohexyl-4'-O-de(2,6-dideoxy-3-O-methyl-α-L-arabino-hexopyranosyl)-5-O-demethyl-25-de(1-methylpropyl)-(9Cl), which comprises the reaction of deglycosidation of 25-cyclohexyl-5-O-demethyl-25-de(1-methylpropyl)avermectin A₁ₐ with a strong acid, in an organic solvent.

16. A process as claimed in Claim 15, wherein the strong acid is an inorganic acid such as sulfuric acid (H₂SO₄), hydrochloric acid (HCl), phosphoric acid (H₃PO₄), or an organic acid, such as trifluoroacetic acid (TFA), p-tolenesulfonic acid (p-TSA), methanesulfonic acid (MSA), or acidic cation resins such as Dowex^{®} 50WX4, Diaion^{®} RCP-160M, Diaion^{®}PK 208LM, and preferably the strong acid is sulfuric acid (H₂SO₄) or p-tolenesulfonic acid (p-TSA).

17. A process as claimed in Claim 15 or Claim 16, wherein the deglycosidation of 25-cyclohexyl-5-O-demethyl-25-de(1-methylpropyl)avermectin A₁ₐ is performed in a mixture of an organic polar solvent and water, said organic polar solvent preferably being acetonitrile or an alcohol in C₁-C₆, such as ethanol.

18. A process as claimed in Claims 15 to 17, wherein the deglycosidation of 25-cyclohexyl-5-O-demethyl-25-de(1-methylpropyl)avermectin A₁ₐ is performed at a temperature of 10-90°C, preferably during a period of 1-8 hours.
